# EUROPEAN PATENT APPLICATION

(11) **EP 4 721 812 A1**
(43) Date of publication of application: **08.04.2026**
(21) Application number: 24205025.0
(22) Date of filing: 07.10.2024
(51) Int. Cl.: A61N 1/39

(54) **ENHANCED CUSTOM OPERATION MODE FOR DEFIBRILLATORS**

(71) Applicant: Koninklijke Philips N.V., 5656 AG Eindhoven (NL)
(72) Inventor: BURGETT, Kevin Scott, Eindhoven (NL); GEHMAN, Stacy Earl, Eindhoven (NL); JORGENSON, Dawn Blilie, 5656AG Eindhoven (NL); LIU, Chenguang, Eindhoven (NL)
(74) Representative: Philips Intellectual Property & Standards

(57) **Abstract**

A defibrillator (200) employing a shock delivery circuit (207) and a defibrillation controller (204) for advising an administration of a cardiopulmonary resuscitation procedure on a heart of a patient by defibrillator (200) in accordance with a rescue protocol including a cardiopulmonary resuscitation protocol and a shock protocol by defibrillator (200). During an ongoing execution of the cardiopulmonary resuscitation protocol by defibrillator (200), the defibrillation controller (200) determines a probable rescue outcome of the heart of the patient or an improbable rescue outcome of the heart of the patient from a shockable cardiac rhythm of an electrocardiogram of the heart of the patient. Based on a determination of the probable rescue outcome of the heart of the patient, the controller (204) pretimely terminates the ongoing execution of the cardiopulmonary resuscitation protocol by the defibrillator (200) and initiates a succeeding execution of the shock protocol by the defibrillator (200) for the shock delivery circuit (207) to deliver the defibrillating shock to the heart of the patient.

## Description

### FIELD OF THE INVENTION

The present disclosure generally relates to a resuscitation rescue effort by a responder or responders of a patient experiencing cardiac arrest. The present disclosure particularly relates to a defibrillator management of the resuscitation rescue effort by a responder or responders of a patient experiencing cardiac arrest to facilitate a successful rescue outcome of the patient.

### BACKGROUND OF THE INVENTION

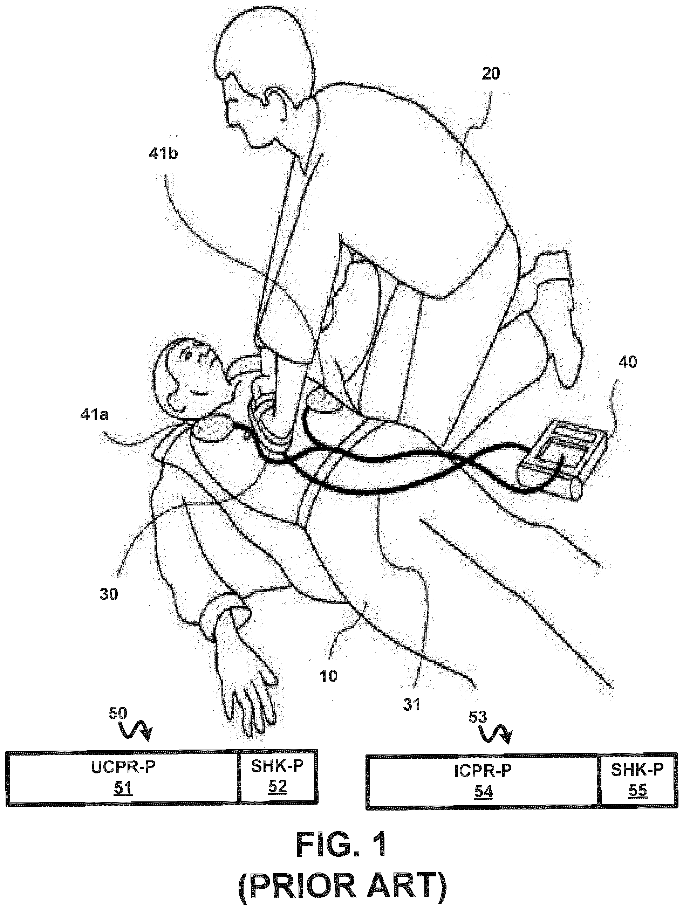

As shown in Fig. 1, a defibrillator 40 is attached to a patient 10 by electrodes 41a and 41b. Defibrillator 40, as known in the art of the present disclosure, can be used to deliver defibrillating shocks to patient 10 when patient 10 is suffering from cardiac arrest. More specifically, defibrillator 40 can deliver a high-voltage impulse to the heart of patient 10 in order to restore organized rhythm and contractile function for the heart of patient 10 when patient 10 is experiencing an arrhythmia (e.g., ventricular fibrillation (VF) or ventricular tachycardia (VT)) that is not accompanied by spontaneous circulation. There are several classes of defibrillators, including manual defibrillators, implantable defibrillators, and automatic/semi-automatic external defibrillators (AEDs). AEDs differ from manual defibrillators in that AEDs can automatically analyze cardiac rhythm(s) of an electrocardiogram (ECG) to determine if defibrillation is necessary. In semi-automatic AED designs, the responder(s) are prompted to press a shock button to deliver the defibrillating shock to the patient when a shock is advised by the AED. The electrodes 41a and 41b are applied across the chest of the patient 10 by a responder 20 as shown in order to acquire an ECG signal from the patient's heart. Defibrillator 40 then analyzes the ECG signal for signs of arrhythmia. If VF or another shockable cardiac rhythm is detected, then defibrillator 40 signals responder 20 that a shock is advised. After the AED detecting VF or another shockable cardiac rhythm and signaling the responder 20 of such detection, then responder 20 presses a shock button on defibrillator 40 to deliver a defibrillating shock in an attempt to resuscitate patient 10.

As shown, a CPR coaching device 30 can be coupled to defibrillator 40 by an electrical cable 31 to provide defibrillator 40 with physiological information obtained by sensors contained in the CPR coaching device 30 as known in the art of the present disclosure. Electrical cable 31 provides power for electronic components in CPR coaching device 30 and couples compression related signals to defibrillator 40 (e.g., compression depth, compression rate, chest release and recoil), whereby the physiological information indicated by the signals can be used to issue audible CPR instructions through the loudspeaker of defibrillator 40.

As known in the art of the present disclosure, defibrillator 40 can be operated in a scheduled operation mode 50 or a custom operation mode 53. In operation, both scheduled operation mode 50 and custom operation mode 53 of defibrillator 40 include a CPR protocol specifying rule(s)/guideline(s) for responder 20 to administer CPR to the heart of patient 10, and a shock protocol for specifying rule(s)/guideline(s) for a delivery (automatic, semi-automatic or manual) of a defibrillating shock to a heart of patient 10.

More particularly, an uninterruptible CPR protocol 51 of the scheduled operation mode 50 is executed over a fixed CPR time period whereby, upon a timely termination of the fixed CPR time period, a scheduled shock protocol 52 of the scheduled operation mode 50 is executed for a delivery of a defibrillating shock in the circumstance that a shockable cardiac rhythm is detected in a corrupt ECG waveform of the heart of patient 10 during the fixed CPR time period of uninterruptible CPR protocol 51.

Scheduled shock protocol 52 can also be executable for a delivery of a defibrillating shock in the circumstance that a shockable cardiac rhythm is not detected in a corrupt ECG waveform of the heart of patient 10 during the fixed CPR time period of uninterruptible CPR protocol 51and a shockable cardiac rhythm is detected in a clean ECG waveform of the heart of patient 10 succeeding the termination of the fixed CPR time period of uninterruptible CPR protocol 51.

Conversely, an interruptible CPR protocol 54 of the custom operation mode 53 is executed over a terminable CPR time period whereby the terminable CPR time period is pre-timely terminated in the circumstance that a shockable cardiac rhythm is detected in a corrupt ECG waveform of the heart of patient 10 during the terminable CPR time period of interruptible CPR protocol 54, and a custom shock protocol 55 of the custom operation mode 53 is immediately prompted for an execution of a delivery of defibrillating shock to the heart of patient 10.

Custom shock protocol 55 can also be executable for a delivery of a defibrillating shock in the circumstances that a shockable cardiac rhythm is not detected in a corrupt ECG waveform of the heart of patient 10 during the terminable CPR time period of interruptible CPR protocol 54 and a shockable cardiac rhythm is detected in a clean ECG waveform of the heart of patient 10 succeeding the timely termination of the terminable CPR time period of interruptible CPR protocol 54.

The defibrillator industry is constantly striving to optimize a systematic operation of a defibrillator to optimize a successful rescue outcome of a patient.

### SUMMARY OF THE INVENTION

The present disclosure improves upon a management of an administration of a cardiopulmonary resuscitation procedure on a heart of a patient in accordance with a rescue protocol including a cardiopulmonary resuscitation protocol and a shock protocol by introducing a principle of terminating an ongoing execution of the cardiopulmonary resuscitation protocol and a commencing a succeeding execution of the shock protocol based on an electrocardiogram of the heart of the patient, during the ongoing execution of the cardiopulmonary resuscitation protocol, indicating a probable rescue outcome of the heart of the patient during the succeeding execution of the shock protocol.

The present disclosure can be embodied as (1) a defibrillation controller, (2) a defibrillator, and (3) a defibrillation method.

Various embodiments of a defibrillation controller of the present disclosure control an administration of a cardiopulmonary resuscitation procedure on a heart of a patient by a defibrillator in accordance with a rescue protocol including a cardiopulmonary resuscitation protocol and a shock protocol.

The various embodiments of the defibrillation controller of the present disclosure employ a non-transitory machine-readable storage medium encoded with instructions for execution by one or more processors to, during an ongoing execution of the cardiopulmonary resuscitation protocol by the defibrillator, determine a probable rescue outcome of the heart of the patient or an improbable rescue outcome of the heart of the patient from a shockable cardiac rhythm of an electrocardiogram of the heart of the patient.

The non-transitory machine-readable storage medium is further encoded with instructions for execution by the processor(s) to, based on a determination of the probable rescue outcome of the heart of the patient during the ongoing execution of the cardiopulmonary resuscitation protocol by the defibrillator, pretimely terminate the ongoing execution of the cardiopulmonary resuscitation protocol by the defibrillator and initiate a succeeding execution of the shock protocol by the defibrillator to deliver the defibrillating shock to the heart of the patient.

The defibrillation controller can be installed within and/or communicatively linked to an external defibrillator (e.g., various types of automatic external defibrillators, semi-automatic defibrillators and monitor/defibrillators).

Various embodiments of a defibrillator of the present disclosure employ a shock delivery circuit and a defibrillation controller for advising an administration of a cardiopulmonary resuscitation procedure on a heart of a patient by the defibrillator in accordance with a rescue protocol including a cardiopulmonary resuscitation protocol and a shock protocol by the defibrillator.

During an ongoing execution of the cardiopulmonary resuscitation protocol by the defibrillator, the defibrillation controller determines a probable rescue outcome of the heart of the patient or an improbable rescue outcome of the heart of the patient from a shockable cardiac rhythm of an electrocardiogram of the heart of the patient.

Based on a determination of the probable rescue outcome of the heart of the patient, the defibrillation controller pretimely terminates the ongoing execution of the cardiopulmonary resuscitation protocol by the defibrillator and initiates a succeeding execution of the shock protocol by the defibrillator for the shock delivery circuit to deliver the defibrillating shock to the heart of the patient.

The defibrillator can be an external defibrillator (e.g., various types of automatic external defibrillators, semi-automatic defibrillators and monitor/defibrillators).

Various exemplary embodiments of a defibrillation method of the present disclosure encompasses an advising by a defibrillator of an administration of a cardiopulmonary resuscitation procedure on a heart of a patient in accordance with a rescue protocol including a cardiopulmonary resuscitation protocol and a shock protocol.

The defibrillation method involves, during an ongoing execution of the cardiopulmonary resuscitation protocol by the defibrillator, a determination of a probable rescue outcome of the heart of the patient or an improbable rescue outcome of the heart of the patient derived from a shockable cardiac rhythm of an electrocardiogram of the heart of the patient.

The defibrillation method further involves, based on a determination of the probable rescue outcome of the heart of the patient, a pretimely termination of the ongoing execution of the cardiopulmonary resuscitation protocol by the defibrillator and an initiation of a succeeding execution of the shock protocol by the defibrillator circuit to deliver the defibrillating shock to the heart of the patient.

The defibrillation method can be implemented by a defibrillation controller installed within and/or communicatively linked to an external defibrillator (e.g., various types of automatic external defibrillators, semi-automatic defibrillators and monitor/defibrillators).

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
Fig. 1 illustrates a cardiopulmonary resuscitation being administered by responder(s) to a heart of a patient as known in the art of the present disclosure;
Fig. 2 illustrates exemplary embodiments of a custom operation mode and a scheduled operation mode of the present disclosure;
Fig. 3 illustrates a flowchart representative of an exemplary embodiment of a CV-Shock advisory method in accordance with the present disclosure;
Fig. 4 illustrates a flowchart representative of a first exemplary embodiment of a custom operation mode of a defibrillation method in accordance with the present disclosure;
Fig. 5A illustrates a flowchart representative of a second exemplary embodiment of a custom operation mode of a defibrillation method in accordance with the present disclosure;
Fig. 5B illustrates a flowchart representative of a vitality trend method in accordance with the present disclosure;
Fig. 6 illustrates an exemplary embodiment of a defibrillator in accordance with the present disclosure;
Fig. 7 illustrates an exemplary embodiment of a defibrillation controller in accordance with the present disclosure; and
Fig. 8 illustrates an exemplary embodiment of an automatic/semi-automatic external defibrillator in accordance with the present disclosure.

### DETAILED DESCRIPTION OF THE PREFERRED EMBODIMENTS

The present disclosure is directed to terminating an ongoing execution of a cardiopulmonary resuscitation protocol and a commencing a succeeding execution of a shock protocol based on an electrocardiogram of the heart of the patient, during the ongoing execution of the cardiopulmonary resuscitation protocol, indicating a probable rescue outcome of the heart of the patient during the succeeding execution of the shock protocol.

For purposes of describing and claiming the present disclosure,
(1) terms of the art of the present disclosure, but not limited to, "defibrillation", "defibrillator", "defibrillating shock", "electrocardiogram (ECG)", "cardiac rhythm", "cardiopulmonary resuscitation (CPR)", "rescue protocol", "CPR protocol", "shock protocol", "scheduled operation mode" and "custom operation mode" are to be interpreted as known in the art of the present disclosure and as exemplary described in the present disclosure;
(2) the term "C-Shock Advisory" broadly encompasses all methods, as known in the art of the present disclosure or hereinafter conceived, for analyzing and classifying a cardiac rhythm of an ECG of a heart of a patient including artifacts as known in the art of the present resulting from an administration of chest compressions to the heart of the patient (i.e., a corrupt ECG) to thereby derive
   (a) a shock decision based upon a determinate classification of the corrupt ECG as having a shockable cardiac rhythm (e.g., a ventricular fibrillation (VF) rhythm or a ventricular tachycardia (VT) rhythm),
   (b) a non-shock decision based upon a determinate classification of the corrupt ECG as having a non-shockable cardiac rhythm (e.g., a pulseless electrical activity rhythm or an asystole rhythm), or
   (c) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(3) the term "C-Shock Delivery" broadly encompasses all methods, as known in the art of the present disclosure or hereinafter conceived, for delivering a defibrillating shock to a heart of a patient corresponding to a shock decision derived by the C-Shock Advisory in accordance with rule(s)/guideline(s) associated with a rescue protocol (e.g., rule(s)/guideline(s) established by the American Heart Association);
(4) the term "F-Shock Advisory" broadly encompasses all methods, as known in the art of the present disclosure or hereinafter conceived, for analyzing and classifying a cardiac rhythm of an ECG of a heart of a patient excluding artifacts as known in the art of the present disclosure resulting from a termination/suspension of an administration of chest compressions to the heart of the patient (i.e., a clean ECG) to thereby derive
   (a) a shock decision based upon a determinate classification of the clean ECG as having a shockable cardiac rhythm (e.g., a ventricular fibrillation (VF) rhythm or a ventricular tachycardia (VT) rhythm),
   (b) a non-shock decision based upon a determinate classification of the clean ECG as having a non-shockable cardiac rhythm (e.g., a pulseless electrical activity rhythm or an asystole rhythm, or
   (c) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(5) the term "F-Shock Delivery" broadly encompasses all methods, as known in the art of the present disclosure or hereinafter conceived, for delivering a defibrillating shock to a heart of a patient corresponding to a shock decision derived by the F-Shock Advisory in accordance with rule(s)/guideline(s) associated with a rescue protocol (e.g., rule(s)/guideline(s) established by the American Heart Association); and
(6) the term "CPR Quality Analysis" broadly encompasses all methods, as known in the art of the present disclosure or hereinafter conceived, for monitoring a quality of CPR being administered to a heart of a patient to derive an assessment of a high quality/compliant CPR or a low quality/non-compliant CPR.

A non-limiting example of a C-Shock Advisory is an Arrythmia Recognition Technology (ART) as known in the art of the present disclosure.

A non-limiting example a F-Shock Advisory is a Patient Analysis System (PAS) as known in the art of the present disclosure.

In practice, for the C-Shock Advisory and the F-Shock Advisory, one or more of the described shock decisions can be omitted and/or additional shock decision(s) can be derived from an analysis and rhythm classification of ECG.

A non-limiting example of CPR Quality Analysis is a monitoring of a depth of compression, a rate of compression and chest release and recoil effective relative to rule(s)/guideline(s) of a rescue protocol (e.g., rule(s)/guideline(s) established by the American Heart Association) to derive an assessment of a high quality/compliant CPR being administered to the heart of the patient or a low quality/non-compliant CPR being administered to the heart of the patient.

An additional non-limiting example of CPR Quality Analysis is an implementation of a physiological sensor/monitor for measuring blood flow, cerebral perfusion and/or myocardial perfusion.

Also for purposes of describing and claiming the present disclosure,
(1) the term "rescue outcome" broadly encompasses a defibrillation of a heart of a patient with or without resuscitation of the patient (e.g., a return of an organized cardiac rhythm with or without a sustained return of spontaneous circulation) and further broadly encompasses a resuscitation of the patient (e.g., a defibrillation of the heart with a sustained return of spontaneous circulation or a survival of the patient with a cerebral performance category 1 or a cerebral performance category 2);
(2) the term "probable rescue outcome" broadly encompasses a probability of a defibrillation of a heart derived, directly or indirectly, from a successful restoration of an organized rhythm and contractile function of a heart from a delivery of a defibrillating shock to the heart of the patient;
(3) the term "improbable rescue outcome" broadly encompasses an improbability of a defibrillation of the heart derived, directly or indirectly, from an unsuccessful restoration of an organized rhythm and contractile function of the heart from a delivery of a defibrillation shock to the heart of the patient;
(4) the term "probable defibrillating shock" broadly encompasses a defibrillating shock delineated as having a probable rescue outcome;
(5) the term "improbable defibrillating shock" broadly encompasses a defibrillating shock delineated as having an improbable rescue outcome;
(6) the term "CV-Shock Advisory" broadly encompasses all methods, as known in the art of the present disclosure or hereinafter conceived, for analyzing and classifying a cardiac rhythm of an ECG of a heart of a patient including artifacts as known in the art of the present resulting from an administration of chest compressions to the heart of the patient (i.e., a corrupt ECG) and for predicting a probable rescue outcome or an improbable rescue outcome to thereby derive
   (a) a CP-shock decision based upon a determinate classification of the corrupt ECG as having a shockable cardiac rhythm (e.g., a ventricular fibrillation (VF) rhythm or a ventricular tachycardia (VT) rhythm) and based further upon a prediction of a probable rescue outcome,
   (b) a CI-shock decision based upon a determinate classification of the corrupt ECG as having a shockable cardiac rhythm (e.g., a ventricular fibrillation (VF) rhythm or a ventricular tachycardia (VT) rhythm) and based further upon a prediction of an improbable rescue outcome,
   (c) a non-shock decision based upon a determinate classification of the corrupt ECG as having a non-shockable cardiac rhythm (e.g., a pulseless electrical activity rhythm or an asystole rhythm, or
   (d) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(7) the term "CP-Shock Delivery" broadly encompasses all methods, as known in the art of the present disclosure or hereinafter conceived, for delivering a defibrillating shock to a heart of a patient corresponding to a CP-shock decision derived by the CV-Shock Advisory in accordance with rule(s)/guideline(s) associated with a rescue protocol (e.g., rule(s)/guideline(s) established by the American Heart Association);
(8) the term "CI-Shock Delivery" broadly encompasses all methods, as known in the art of the present disclosure or hereinafter conceived, for delivering a defibrillating shock to a heart of a patient corresponding to a CI-shock decision derived by the CV-Shock Advisory in accordance with rule(s)/guideline(s) associated with a rescue (e.g., rule(s)/guideline(s) established by the American Heart Association);
(9) the term "FV-Shock Advisory" broadly encompasses all methods, as known in the art of the present disclosure or hereinafter conceived, for analyzing and classifying a cardiac rhythm of an ECG of a heart of a patient excluding artifacts as known in the art of the present disclosure resulting from an administration of chest compressions to the heart of the patient (i.e., a clean ECG) and for predicting a probable rescue outcome or an improbable rescue outcome to thereby derive
   (a) a FP-shock decision based upon a determinate classification of the clean ECG as having a shockable cardiac rhythm (e.g., a ventricular fibrillation (VF) rhythm or a ventricular tachycardia (VT) rhythm) and upon a prediction of a probable rescue outcome,
   (b) a FI-shock decision based upon a determinate classification of the clean ECG as having a shockable cardiac rhythm (e.g., a ventricular fibrillation (VF) rhythm or a ventricular tachycardia (VT) rhythm) and upon a prediction of an improbable rescue outcome,
   (c) a non-shock decision based upon a determinate classification of the clean ECG as having a non-shockable cardiac rhythm (e.g., a pulseless electrical activity rhythm or an asystole rhythm, or
   (d) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(10) the term "FP-Shock Delivery" broadly encompasses all methods, as known in the art of the present disclosure or hereinafter conceived, for delivering a defibrillating shock to a heart of a patient corresponding to a FP-shock decision derived by the FV-Shock Advisory in accordance with rule(s)/guideline(s) associated with a rescue protocol (e.g., rule(s)/guideline(s) established by the American Heart Association); and
(11) the term "FI-Shock Delivery" broadly encompasses all methods, as known in the art of the present disclosure or hereinafter conceived, for delivering a defibrillating shock to a heart of a patient corresponding to a FI-shock decision derived by the FV-Shock Advisory in accordance with rule(s)/guideline(s) associated with a rescue protocol (e.g., rule(s)/guideline(s) established by the American Heart Association).

A non-limiting example of a FV-Shock Advisory is a Patient Analysis System (PAS) as known in the art of the present disclosure incorporating a measurement of a vitality of a shockable cardiac rhythm as known in the art of the present disclosure (e.g., a measurement of an amplitude/frequency of the shockable cardiac rhythm relative to a sensitivity/specificity based threshold delineating between a probable rescue outcome or an improbable rescue outcome).

A non-limiting example of a CV-Shock Advisory is an Arrythmia Recognition Technology (ART) as known in the art of the present disclosure incorporating a measurement of a vitality of a shockable cardiac rhythm as known in the art of the present disclosure (e.g., a measurement of an amplitude/frequency of the shockable cardiac rhythm relative to a sensitivity/specificity based threshold delineating between a probable rescue outcome or an improbable rescue outcome).

In practice, for the CV-Shock Advisory and the FV-Shock Advisory, one or more of the described shock decisions can be omitted and/or additional shock decision(s) can be derived from an analysis and classification of a cardiac rhythm of an ECG.

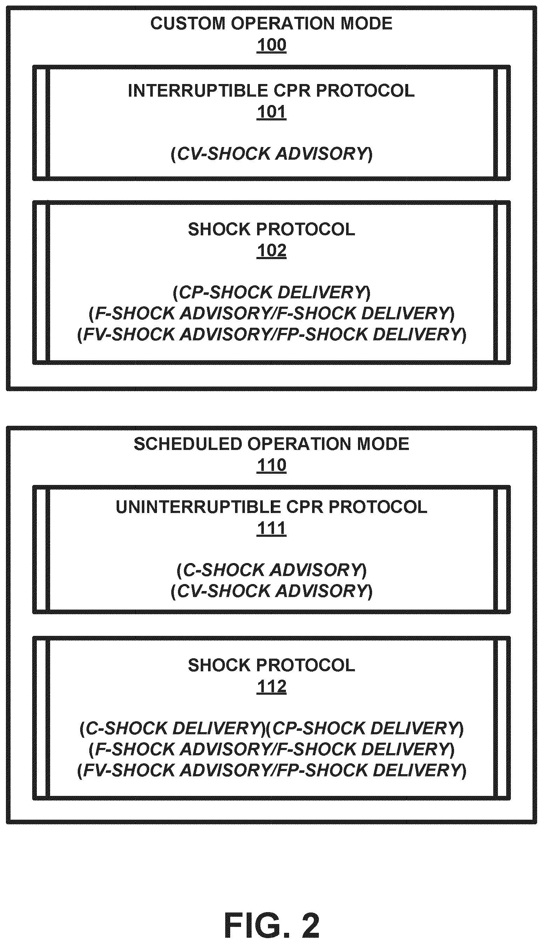

To facilitate an understanding of the present disclosure, the following description of Figs. 2-5B describes and teaches exemplary embodiments of methods in accordance with the present disclosure. From the description of Figs. 2-5B, those having ordinary skill in the art of the present disclosure will appreciate how to apply the present disclosure to make and use additional embodiments of methods in accordance with the present disclosure.

Fig. 2 illustrates an exemplary embodiment of a custom operation mode 100 of the present disclosure and an exemplary embodiment of a scheduled operation mode 110 of the present disclosure.

Referring to Fig. 2, custom operation mode 100 includes an interruptible CPR protocol 101 specifying rule(s)/guideline(s) for a guidance by a defibrillator of an administration of a cardiopulmonary resuscitation to a heart of patient over a terminable CPR time period of the interruptible CPR protocol (e.g., > two (2) minutes).

To this end, interruptible CPR protocol 101 employs a CV-Shock Advisory to ascertain whether to pretimely terminate an execution of the interruptible CPR protocol 101 by the defibrillator based on a CP shock decision by the CV-Shock Advisory, or to timely terminate an execution of the interruptible CPR protocol 101 by the defibrillator based on a CI-shock decision, no-shock decision or an undecided shock decision by the CV-Shock Advisory.

Custom operation mode 100 further includes a shock protocol 102 specifying rule(s)/guideline(s) for a conditional delivery of a defibrillating shock to the heart of the patient by the defibrillator subsequent to a pretimely termination or a timely termination of an execution of interruptible CPR protocol 101 by the defibrillator.

To this end, shock protocol 102 (a) employs a CP-Shock Delivery to deliver a probable defibrillating shock to a heart of a patient by the defibrillator in response to a pre-timely termination of an execution of interruptible CPR protocol 101 by the defibrillator and a CP-shock decision by the CV-Shock Advisory, and (b) optionally employs a CI-Shock Delivery to deliver an improbable defibrillating shock to a heart of a patient by the defibrillator in response to a timely termination of an execution of interruptible CPR protocol 101 by the defibrillator and a CI-shock decision by the CV-Shock Advisory.

Shock protocol 102 further employs a F-Shock Advisory and a F-Shock Delivery to deliver a defibrillating shock to a heart of a patient by the defibrillator in response to a timely termination of an execution of interruptible CPR protocol 101 by the defibrillator and a F-shock decision by the F-Shock Advisory.

Concurrently or alternatively, shock protocol 102 employs a FV-Shock Advisory and a FP-Shock Delivery to deliver a probable defibrillating shock to a heart of a patient by the defibrillator in response to a timely termination of an execution of the CPR protocol 101 by the defibrillator and a FP-shock decision by FV-Shock Advisory.

Still referring to Fig. 2, scheduled operation mode 110 includes an uninterruptible CPR protocol 111 specifying rule(s)/guideline(s) for a guidance by a defibrillator of an administration of a cardiopulmonary resuscitation to a heart of patient over a fixed CPR time period of the uninterruptible CPR protocol 111 (e.g., two (2) minutes).

To this end, uninterruptible CPR protocol 111 can employ a C-Shock Advisory to render a C-shock decision, a no-shock decision, an undecided shock decision by the C-Shock Advisory during the fixed CPR time period of uninterruptible CPR protocol 111.

Alternatively or concurrently, CPR protocol 111 can employ a CV-Shock Advisory to render a CP-shock decision, a CI-shock decision, a no-shock decision or an undecided shock decision by the CV-Shock Advisory during the fixed CPR time period of uninterruptible CPR protocol 111.

Scheduled operation mode 110 further includes a shock protocol 112 specifying rule(s)/guideline(s) for a conditional delivery of a defibrillating shock to the heart of the patient by the defibrillator subsequent to a timely termination of an execution of the CPR protocol 111 by the defibrillator.

To this end, shock protocol 102 can (a) employ a C-Shock Delivery to deliver a defibrillating shock to a heart of a patient by the defibrillator in response to a timely termination of an execution of uninterruptible CPR protocol 111 by the defibrillator and a C-shock decision by the CV-Shock Advisory, (b) employ a CP-Shock Delivery to deliver a probable defibrillating shock to a heart of a patient by the defibrillator in response to a timely termination of an execution of uninterruptible CPR protocol 111 by the defibrillator and a CP-shock decision by the CV-Shock Advisory, and (c) optionally employ a CI-Shock Delivery to deliver an improbable defibrillating shock to a heart of a patient by the defibrillator in response to a timely termination of an execution of interruptible CPR protocol 101 by the defibrillator and a CI-shock decision by the CV-Shock Advisory.

Shock protocol 102 further employs a F-Shock Advisory and a F-Shock Delivery to deliver a defibrillating shock to a heart of a patient by the defibrillator in response to a timely termination of an execution of interruptible CPR protocol 101 by the defibrillator and a F-shock decision by the F-Shock Advisory.

Concurrently or alternatively, shock protocol 102 employs a FV-Shock Advisory and a FP-Shock Delivery to deliver a probable defibrillating shock to a heart of a patient by the defibrillator in response to a timely termination of an execution of the CPR protocol 101 by the defibrillator and a FP-shock decision by FV-Shock Advisory.

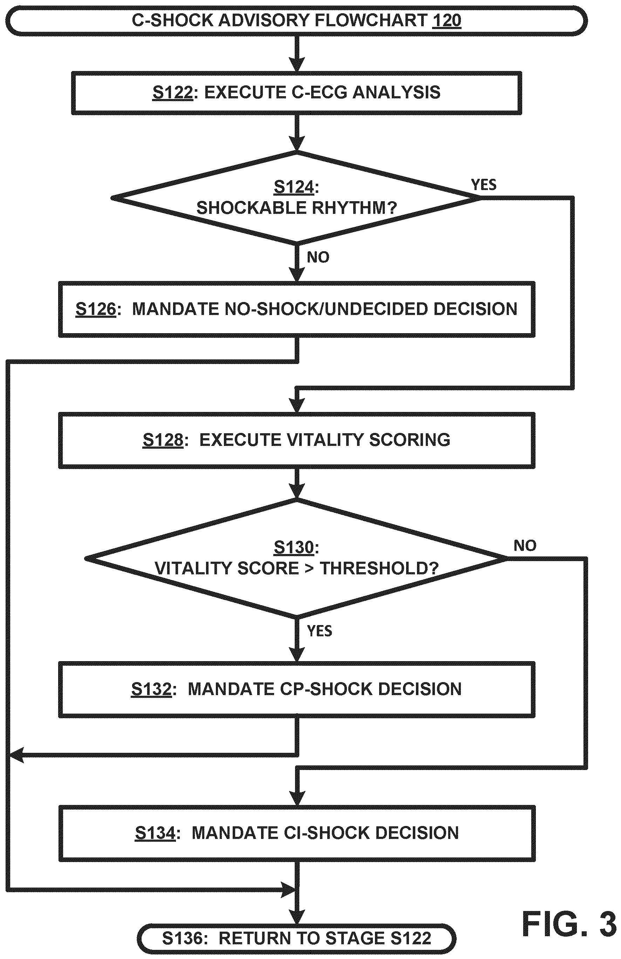

Fig. 3 illustrates a flowchart 120 representative of a CV-Shock Advisory method of the present disclosure for a custom operation mode of the present disclosure.

Referring to Fig. 3, a stage S122 of flowchart 120 encompasses an execution of a C-ECG Analysis for analyzing an ECG of a heart of a patient including artifacts, as known in the art of the present or herein conceived, resulting from an administration of chest compressions to the heart of the patient (i.e., a corrupt ECG), and rhythm classifying the ECG as a shockable cardiac rhythm (e.g., a ventricular fibrillation (VF) rhythm or a ventricular tachycardia (VT) rhythm), a non-shockable cardiac rhythm (e.g., a pulseless electrical activity rhythm or an asystole rhythm) or an undecided cardiac rhythm.

If the C-ECG is determined to be classified as a non-shockable cardiac rhythm or undecided cardiac rhythm during a stage S124 of flowchart 120, then a stage S126 of flowchart 120 encompasses a no-shock/undecided decision and a return to stage S122 for further analysis and rhythm classification of the C-ECG.

If the ECG is determined to be classified as a shockable cardiac rhythm during stage S124, then a stage S128 of flowchart 120 encompasses a computation of vitality score(X) of the shockable cardiac rhythm whereby if the vitality score(X) is greater than a threshold of stage S130 of flowchart 120, then flowchart 120 proceeds to a stage S132 to mandate a CP-Shock decision and return to stage 5122 for further analysis and rhythm classification of the C-ECG and whereby if the vitality score(X) is equal to or less than the threshold of stage S130 of flowchart 120, then flowchart 120 proceeds to a stage S134 to mandate a CI-Shock decision, and returns to stage S122 for further analysis and rhythm classification of the ECG.

In practice, stages 128 and 130 involve a measurement of an energy, amplitude and/or frequency of the shockable cardiac rhythm relative to a sensitivity/specificity based threshold delineating between a probable rescue outcome or an improbable rescue outcome.

In one exemplary embodiment, the vitality score is calculated by first applying bandpass filters to the ECG to remove CPR artifacts from the signal as known in the art of the present disclosure, and secondly calculating a mean magnitude of a first difference of the bandpass filtered ECG signal.

Alternatively, the vitality score can be calculated from a power signal of the filtered ECG, such as, for example, the vitality score can be calculated as the mean of the power signal. Some variations of this calculation method include using the median, 10% trimmed mean, 20% trimmed mean, or 30% trimmed mean of the power signal as opposed to the mean amplitude.

In practice, the two aforementioned calculation methods can be combined by a dimensionality reduction method as known in the art of the present disclosure, such as, for example, a principal component analysis can be employed to combine the information from the various measurement into one value of the VF score.

Also in practice, the vitality scoring can incorporate features which capture the entropy of the ECG signal, and can also incorporate dichotomous variables, such as, for example prior return of resuscitation (ROR), age, and sex in its estimation of rescue outcome.

Still referring to Fig. 3, flowchart 120 involves a continuous calculation of the vitality scoring while CPR is ongoing to the patient, and the vitality scoring and the threshold to predict a particular shock outcome (e.g., ROR, ROSC and survival).

In practice, the threshold can be adapted according to AED use time and the vitality scoring for the patient in prior CPR protocols within the same resuscitation.

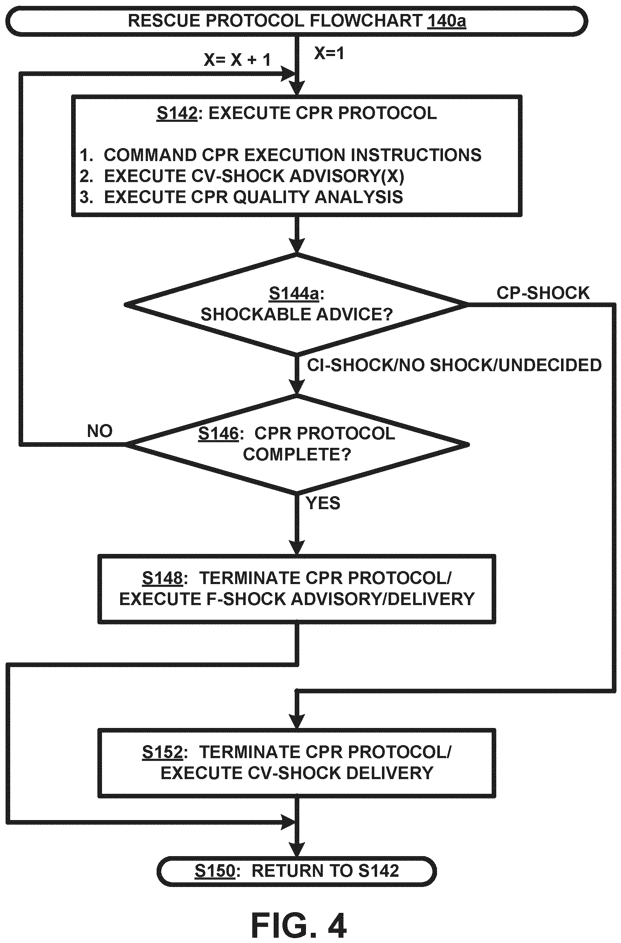

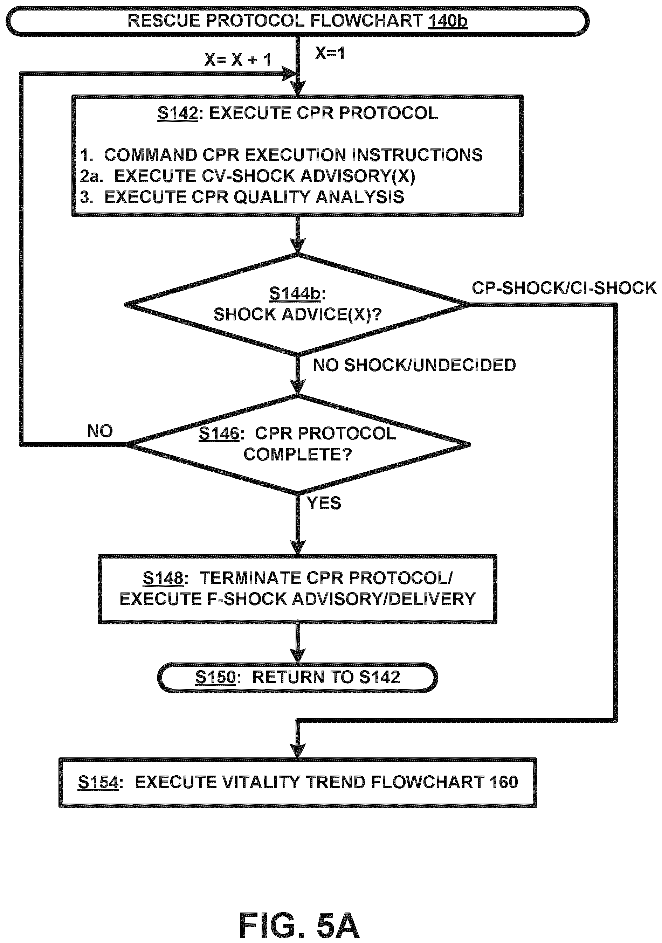

Also in practice, a current indication of a probable rescue outcome via the CP-Shock decision of stage S132 is utilized in a rescue protocol flowchart 140a of Fig. 4, and a trending indication of a probable rescue outcome via a comparison of two CP-Shock decisions of stage S132 is utilized in a rescue protocol flowchart 140b of Fig. 5A.

Referring to Fig. 4, a stage S142 of rescue protocol flowchart 140a encompasses an execution of an interruptible CPR protocol including (1) a communication of CPR execution instructions to responder(s) (not shown) or a CPR mechanical device (not shown) for administering CPR to the heart of the patient, (2) an execution of a CV-Shock Advisory of Fig. 3 to render a "CP-Shock" decision, a CI-Shock" decision or a "No Shock/Undecided" decision, and (3) an optional monitoring of CPR Quality Analysis of the CPR being administered by the responder or the CPR mechanical device.

A stage S144a of flowchart 140a determines if the CV-Shock Advisory of stage S142 is "CP-Shock" decision, a "CI-Shock' decision or "No Shock/Undecided" decision.

If the CV-Shock Advisory is a "CP-Shock" decision for stage S144a, then flowchart 140a proceeds to a stage S150 to pretimely terminate the execution of the interruptible CPR protocol of stage S142 and execute a shock protocol including (1) issuing CPR interruption instructions to the responder (not shown) or CPR mechanical device (not shown), and (2) prompting and monitoring a CP-Shock Delivery of the defibrillating shock as known in the art of the present disclosure. Thereafter, a stage S152 of flowchart 140a returns to stage S142.

If the CV-Shock Advisory is a "CI-Shock" decision or a "No Shock/Undecided" decision for stage S144a, then flowchart 140a proceeds to a stage S146 to determine if the terminable CPR time period of the interruptible CPR protocol of stage S142 has timely expired (e.g., two minutes).

If the terminable CPR time period the interruptible CPR protocol has not timely expired, then flowchart 140a returns to stage S142.

If the terminable CPR time period the interruptible CPR protocol has timely expired, then flowchart 140a proceeds to a stage S148 of flowchart S140a to timely terminate the execution of the interruptible CPR protocol of stage S142 and execute the shock protocol including a communication of CPR termination instructions to responder(s) (not shown) or a CPR mechanical device (not shown).

The execution of the shock protocol of stage S148 further includes (1) prompting and monitoring a CI-Shock Delivery of the defibrillating shock as known in the art of the present disclosure in response to a "CI-Shock decision", and (2) an execution of F-Shock Advisory and F-Shock delivery as shown for a conditional delivery of a defibrillating shock to the heart of the patient, or alternatively an execution of a FV-Shock Advisory and FP-Shock delivery for a conditional delivery of a probable defibrillating shock to the heart of the patient based on a probable rescue outcome determined by a version of flowchart 120 of Fig. 3 corresponding to a clean ECG of stage S122, a FP-shock decision mandate of stage 132 and FI-shock decision mandate of stage S134.

Referring to Fig. 5A, a stage S142 of rescue protocol flowchart 140b encompasses 140a encompasses an execution of an interruptible CPR protocol including (1) a communication of CPR execution instructions to responder(s) (not shown) or a CPR mechanical device (not shown) for administering CPR to the heart of the patient, (2) an execution of a CV-Shock Advisory of Fig. 3 to render a "CP-Shock" decision, a CI-Shock" decision or a "No Shock/Undecided" decision and (3) an optional monitoring of CPR Quality Analysis of the CPR being administered by the responder or the CPR mechanical device.

A stage S144b of flowchart 140b determines if the CV-Shock Advisory of stage S142 is "CP-Shock" decision, a "CI-Shock' decision or "No Shock/Undecided" decision.

If the CV-Shock Advisory is a "CP-Shock" decision for stage S144b, then flowchart 140b proceeds to a stage S150 to pretimely terminate the execution of the interruptible CPR protocol of stage S142 and execute a shock protocol including (1) issuing CPR interruption instructions to the responder (not shown) or CPR mechanical device (not shown), and (2) prompting and monitoring a CP-Shock Delivery of the defibrillating shock as known in the art of the present disclosure. Thereafter, a stage S152 of flowchart 140b returns to stage S142.

If the CV-Shock Advisory is a "No Shock/Undecided" decision for stage S144b, then flowchart 140b proceeds to a stage S146 to determine if the terminable CPR time period of the interruptible CPR protocol of stage S142 has timely expired (e.g., two minutes).

If the terminable CPR time period the interruptible CPR protocol has not timely expired, then flowchart 140a returns to stage S142.

If the terminable CPR time period the interruptible CPR protocol has timely expired, then flowchart 140a proceeds to a stage S148 of flowchart S140b to timely terminate the execution of the interruptible CPR protocol of stage S142 and execute the shock protocol including a communication of CPR termination instructions to responder(s) (not shown) or a CPR mechanical device (not shown).

The execution of the shock protocol of stage S148 further includes an execution of F-Shock Advisory and F-Shock delivery as shown for a conditional delivery of a defibrillating shock to the heart of the patient, or alternatively an execution of a FV-Shock Advisory and FP-Shock delivery for a conditional delivery of a probable defibrillating shock to the heart of the patient based on a probable rescue outcome determined by a version of flowchart 120 of Fig. 3 corresponding to a clean ECG of stage S122, a FP-shock decision mandate of stage 132 and FI-shock decision mandate of stage S134.

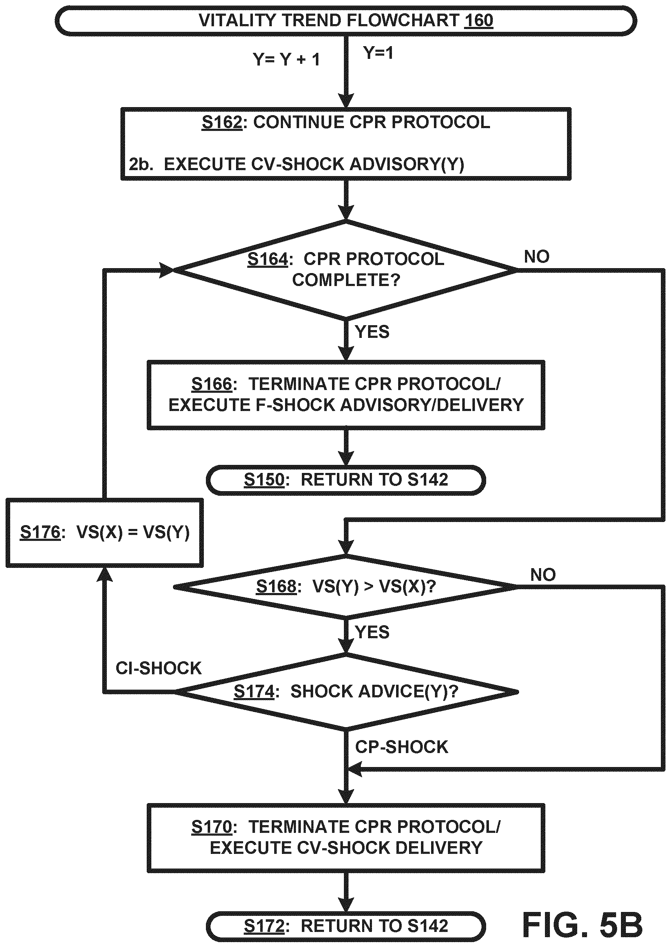

Still referring to Fig. 5A, if the CV-Shock Advisory is a "CI-Shock" decision for stage S144b, then flowchart 140b proceeds to a stage S154 to execute a vitality trend flowchart 160 of Fig. 5B including an acquisition of a current vitality score(X) of flowchart 130.

Referring to Fig. 5B, a stage S162 of flowchart 160 continues with the execution of interruptible CPR protocol including an execution of a CV-Shock Advisory (y) including a computation of a vitality score(Y).

If the terminable CPR time period of interruptible CPR protocol has timely expired as determined by a stage S164 of flowchart 160, then flowchart 160 proceeds to a stage S166 to timely terminate the execution of the interruptible CPR protocol of stage S162 and execute the shock protocol including a communication of CPR termination instructions to responder(s) (not shown) or a CPR mechanical device (not shown).

The execution of the shock protocol of stage S166 further includes an execution of F-Shock Advisory and F-Shock delivery as shown for a conditional delivery of a defibrillating shock to the heart of the patient, or alternatively an execution of a FV-Shock Advisory and FP-Shock delivery for a conditional delivery of a probable defibrillating shock to the heart of the patient based on a probable rescue outcome determined by a version of flowchart 120 of Fig. 3 corresponding to a clean ECG of stage S122, a FP-shock decision mandate of stage 132 and FI-shock decision mandate of stage S134.

Still referring to Fig. 5B, if the terminable CPR time period of interruptible CPR protocol has not timely expired as determined by stage S164 of flowchart 160, then flowchart 160 proceeds to a stage S168 of flowchart 160 to compare the vitality score(Y) of stage S162 to vitality score(X) of stage S154 of flowchart 140b.

If the vitality score(Y) is less than or equal to the vitality score(X), the flowchart 160 proceeds to a stage 170 to pretimely terminate the execution of the interruptible CPR protocol of stage S162 and to execute the shock protocol including (1) issuing CPR interruption instructions to the responder (not shown) or CPR mechanical device (not shown), and (2) prompting and monitoring a CP-Shock Delivery of the defibrillating shock as known in the art of the present disclosure.

If the vitality score(Y) is greater than the vitality score(X) and the CV-Shock advisory(Y) of stage S162 was a "CP-Shock" decision, the flowchart 160 proceeds to stage 170 to pretimely terminate the execution of the CPR protocol of stage S162 and execute the shock protocol including (1) issuing CPR interruption instructions to the responder (not shown) or CPR mechanical device (not shown), and (2) prompting and monitoring a CP-Shock Delivery of the defibrillating shock as known in the art of the present disclosure.

Otherwise, if the vitality score(Y) is greater than the vitality score(X) and the CV-Shock advisory(Y) of stage S162 was a "CI-Shock" decision, the flowchart 160 proceeds to stage 176 to equate the vitality score(X) to the vitality score (Y) and returns to stage S162.

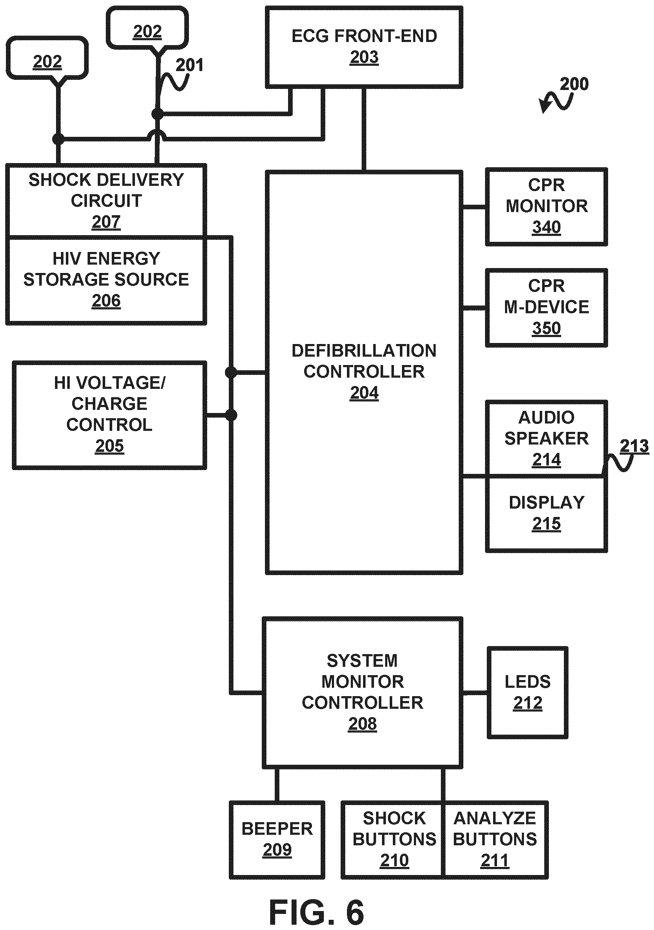

To facilitate a further understanding of the present disclosure, the following description of Figs. 6-8 teaches exemplary embodiments of devices and systems in accordance with the present disclosure. From the description of Figs. 6-8, those having ordinary skill in the art of the present disclosure will appreciate how to apply the present disclosure to make and use additional embodiments of devices and systems in accordance with the present disclosure.

The afore-described methods can be implemented in a medical device, such as, for example, a defibrillator (e.g., an external defibrillator). Fig. 6 is a functional block diagram of an external defibrillator 204 according to the one embodiment of the present disclosure. Defibrillator 204 is configured as an AED that is intended for use during a cardiac rescue which includes CPR. It is designed for small physical size, light weight, and relatively simple responder(s) interface capable of being operated by personnel without high training levels or who otherwise would use the defibrillator 204 only infrequently. Although the present embodiment of the invention is described with respect to application in an AED, other embodiments include application in different types of defibrillators, for example, manual defibrillators, fully automatic defibrillators, and paramedic or clinical defibrillator/monitors.

Defibrillator 204 receives an input 214 of an ECG signal from, for example, two or more electrodes 202 that are connected to a patient. An ECG front end circuit 203 is in electrical communication with the input 2141 via a connector plug and socket or the like. The ECG front end circuit 203 operates to amplify, buffer, filter and optionally digitize an electrical ECG signal generated by the patient's heart to produce a stream of digitized ECG samples. The digitized ECG samples are provided to a controller 204, which can be a processor that combines a DSP and ARM processor. One exemplary controller is the family of Applications Processors manufactured by Texas Instruments Incorporated Inc. In one embodiment of the apparatus, the DSP conducts all of the previously described filtering under the ART protocol, and then passes the multiple streams of filtered ECG data to the ARM processor. The ARM buffers the stream of digitized ECG signal data into segments (buffers) corresponding to a predetermined time. The ARM performs an outcomes analysis on the filtered ECG data to detect VF, shockable VT or other shockable cardiac rhythms. In accordance with the present disclosure, the ARM uses the outcomes analysis to determine a treatment regimen which is most beneficial to the patient. These controller 204 portions of the DSP and ARM thus operate together as an ECG analyzer. Of course, the scope of the present disclosure is not limited to a particular DSP/ARM configuration. The foregoing and following functions can be equivalently implemented in a single processor or distributed among multiple processors.

The ECG analyzer incorporates an analysis algorithm that can determine a shockable cardiac rhythm in the presence of CPR-related signal noise artifact with a defined sensitivity and specificity. The accuracy of the ECG analyzer is sufficient to safely and effectively assess the cardiac state of the input signal in the presence of CPR compressions noise. One such analysis algorithm is ART as described previously.

If the ECG analyzer determines a shockable cardiac rhythm in combination with the determination of a treatment regimen that indicates the need for a defibrillating shock, then controller 204, responsive to the output of the ECG analyzer, sends a signal to a HV (high voltage) charging circuit 2145 to charge a HV energy storage source 206 in preparation for delivering a shock. When the HV energy storage source 206 is fully charged, controller 204 directs a shock button 210 to begin flashing to re-direct the attention of the responder(s) from the task of providing CPR compressions to the task of delivering electrotherapy.

As will be described in more detail, controller 204 can initiate the preparation for a defibrillating shock immediately upon detection of a shockable cardiac rhythm, i.e. in a continuous mode of operation, and issue instructions to interrupt of CPR compressions for electrotherapy as soon as the device is armed. Alternatively, the controller 204 can initiate preparation for a defibrillating shock preceding the end of a predetermined period of CPR compressions, and can instruct the immediate delivery of electrotherapy simultaneously with the end of the predetermined period. This last mode is called a scheduled operation mode.

In either continuous or scheduled operation mode, controller 204 controls the responder(s) interface 213 to issue aural prompts to terminate CPR and press the shock button to deliver a defibrillating shock. These prompts should be issued together and in quick order so that delay between stopping CPR and pressing the shock button is minimized. The responder(s) interface 213 should similarly issue an aural prompt via audio speaker 214 to resume CPR as soon as possible after the controller 204 senses that a defibrillating shock has been delivered, e.g. by sensing the button press, current flow from the HV storage circuit, etc. corresponding visual prompts can be issued simultaneously with the aural prompts.

When the responder(s) presses the shock button 210, a defibrillating shock is delivered from HV energy storage source 206 through a Shock delivery circuit 207. In a preferred embodiment, Shock delivery circuit 207 is electrically connected via an output of the AED to the same electrodes 202 which receive the raw ECG signal.

Controller 204 also provides control of the responder(s) interface (UI) output functions in the device. The responder(s) interface 213 is the primary means for guiding the responder(s) through the progress of the cardiac rescue protocol, and so includes at least one of an aural instruction output and a visual display. In particular, responder(s) interface 213 may comprise an audio speaker 214 to issue an aural verbal or signal prompt to the responder(s) regarding a state of the rescue, an instruction as to a next step to be taken in the rescue, or regarding instructions responsive the determined shockable cardiac rhythm. Responder(s) interface 213 may also convey audible information via a beeper 209. Responder(s) interface 213 may also provide visual text or graphical indications on a display 215. Responder(s) interface 213 may also convey visual information via flashing light LED 212, which may illuminate adjacent graphics or buttons to be pressed. Preferably, controller 204 controls the responder(s) interface such that each of these cues is provided in a manner that optimizes the desired response of the responder(s).

Audible and visual cues pertaining to the same information need not be issued simultaneously if one or the other cue may detract from the desired response. For example, controller 204 may control the charging circuit to fully charge the HV storage source to the armed state preceding issuing any instructions at all. Alternatively, controller 204 may drive the responder(s) interface to indicate a determination of a shockable cardiac rhythm on visual display 215 preceding issuing related aural instructions on speaker 214. And again, controller 204 may drive the responder(s) interface to indicate the state of the HV charging circuit preceding issuing related aural instructions on speaker 214.

Software instructions for operating controller 204 are disposed in an onboard memory. Instructions in non-volatile memory may include the algorithm for the ART algorithm, the algorithm for PAS, instructions for a CPR rescue protocol that includes a period for providing CPR compressions, UI configurations for multiple responder(s) types, and the like. Volatile memory may include software-embodied records of device self-tests, device operating data, and rescue event audio and visual recordings.

Other optional features of the defibrillator shown in Fig. 5 include a system monitor controller which receives signals from various Buttons (e.g. Power On, Shock) and provides signals for the beeper and LED lights. State changes of the buttons and sensors are transmitted back to the controller 204 through a communications interface. This feature enables very low-power standby operations with wake-up sensing by means of the button actuation and readiness status outputs.

Furthermore, additional optional features include communication links with a CPR monitor 340 or a CPR mechanical device 350 as known in the art of the present disclosure.

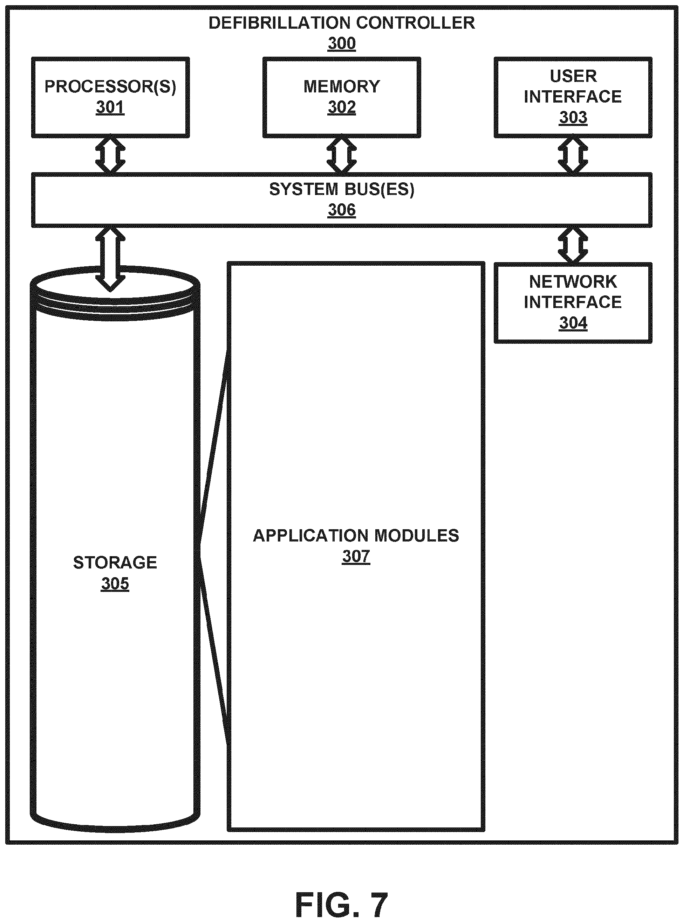

Referring to Fig. 7, shown is an exemplary embodiment of controller 300 that includes one or more processor(s) 301, memory 302, a responder(s) interface 303, a network interface 304, and a storage 305 interconnected via one or more system bus(es) 306.

Each processor 301 can be any hardware device, as known in the art of the present disclosure or hereinafter conceived, capable of executing instructions stored in memory 302 or storage or otherwise processing data. In a non-limiting example, the processor(s) 301 can include a microprocessor, field programmable gate array (FPGA), application-specific integrated circuit (ASIC), or other similar devices.

The memory 302 can include various memories, as known in the art of the present disclosure or hereinafter conceived, including, but not limited to, L1, L2, or L3 cache or system memory. In a non-limiting example, the memory 302 can include static random access memory (SRAM), dynamic RAM (DRAM), flash memory, read only memory (ROM), or other similar memory devices.

In practice, controller 300 also provides control of the responder(s) interface (UI) output functions. Specifically, responder(s) interface 303 is the primary means for guiding the responder through the protocols of the present disclosure, and so includes at least one of an aural instruction output and a visual display. In particular, responder(s) interface 303 may comprise an audio speaker to issue an aural verbal or signal prompt to the responder regarding a state of the rescue, an instruction as to a next step to be taken in the rescue, or regarding instructions responsive to an execution of a particular protocol (e.g., administering CPR and/or delivering a drug). Responder(s) interface 303 can also convey audible information via a beeper. Responder(s) interface 303 can also provide visual text or graphical indications on a display. Responder(s) interface 303 can also convey visual information via a flashing light LED, which may illuminate adjacent graphics or buttons to be pressed. Preferably, controller 300 controls the responder(s) interface 301 such that each of these cues is provided in a manner that optimizes the desired response of the responder in the execution of protocols of the present disclosure. Still referring to Fig. 7, network interface 304 can include one or more devices, as known in the art of the present disclosure or hereinafter conceived, for enabling communication with other components of defibrillator (defibrillator 200 of Fig. 6) or another device, particularly a mechanical CPR device or a CPR coaching device, as known in the art of the present disclosure or hereinafter conceived, in the administration of CPR/chest compression to a patient in accordance with the protocols of the present disclosure and/or in the acquisition of CPR data indicative of the quality of CPR being administered to the patient.

In a non-limiting example, the network interface 304 can include a network interface card (NIC) configured to communicate according to the Ethernet protocol. Additionally, the network interface 414 may implement a TCP/IP stack for communication according to the TCP/IP protocols. Various alternative or additional hardware or configurations for the network interface 304 will be apparent.

The storage 305 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 305 can store instructions for execution by the processor(s) 301 or data upon with the processor(s) 301 may operate. For example, the storage 305 may store a base operating system for controlling various basic operations of the hardware.

The storage 305 can also store an application modules 307 in the form of executable software/firmware for implementing the methods of the present disclosure as previously described in the present disclosure.

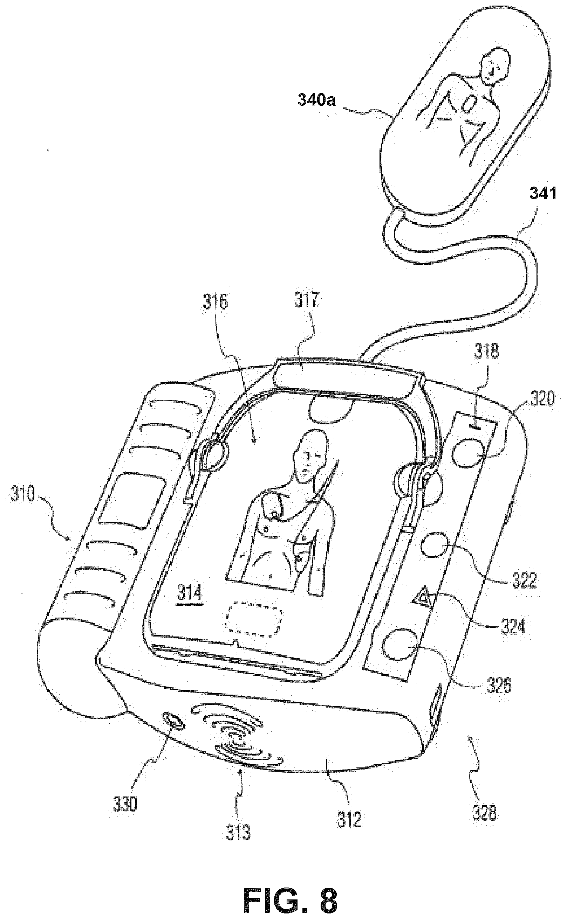

Fig. 8 illustrates the CPR coaching device 100 coupled through cable 130 to the defibrillator 310. The defibrillator 310 represents a semi-automatic external defibrillator (AED). However, other types of defibrillators can be used as well. The AED 310 is housed in a rugged polymeric case 312 which protects the electronic circuitry inside the case, which was previously described with reference to Fig. 5, and also protects the responder 220 from shocks. Attached to the case 312 by electrical leads are a pair of electrodes 316. The electrodes 316 are housed in a cartridge 314 located in a recess on the top side of the AED 310. The electrode pads are accessed for use by pulling up on a handle 317 which allows removal of a plastic cover over the electrodes 316. The responder(s) interface is on the right side of the AED 310. A small ready light 318 informs the responder 220 of the readiness of the AED 310. In this embodiment the ready light blinks after the AED 310 has been properly set up and is ready for use. The ready light is on constantly when the AED 310 is in use, and the ready light is off or flashes in an alerting color when the AED 310 needs attention.

Below the ready light is an on/off button 320. The on/off button is pressed to turn on the AED 310 for use. To turn off the AED 310 the responder 220 holds the on/off button down for one second or more. An information button 322 flashes when information is available for the responder 220. The responder 220 depresses the information button to access the available information, which is then presented as an audible message. A caution light 324 blinks when the AED 310 is acquiring heartbeat information from the patient 210 and lights continuously when a shock is advised, alerting the responder 220 and others that no one should be touching the patient 210 during these times. A shock button 326 is depressed to deliver a shock after the AED 310 informs the responder 220 that a shock is advised. An infrared port 328 on the side of the AED 310 is used to transfer data between the AED 310 and a computer. This data port finds used after the patient 210 has been rescued and a physician desires to have the AED 310 event data downloaded to his or her computer for detailed analysis. A speaker 313 provides voice instructions to the responder 220 to guide the responder 220 through the use of the AED 310 to treat the patient 210. A beeper 330 is provided which "chirps" when the AED 310 needs attention such as electrode pad replacement or a new battery. The beeper can also be used as a metronome tone which chirps at the appropriate rate of CPR chest compressions.

In another embodiment the CPR coaching device includes ECG electrodes on the body-contacting surface of the device for the sensing of the patient's ECG signal. The ECG signal detected by the CPR coaching device is coupled to the ECG front end circuit 202 for processing. In one implementation the CPR coaching device of this embodiment is applied to the patient's chest before the usual defibrillator electrodes are unwrapped and applied. The ECG sensor on the coaching device can thereby give the defibrillator a "quick look" at the patient's ECG waveform. For instance, if the ECG signals is sensed and processed to determine that the patient exhibits a viable ECG signal, the defibrillator can alert the responder that defibrillation is not advised for the patient. The responder does not need to unwrap and apply the defibrillation electrodes to the patient and another form of therapy can be recommended by the defibrillator such as CPR.

From the description of Figs. 1-8 herein, those having ordinary skill in the art will appreciate the numerous benefits of the present disclosure including, but not limited to, improving upon a management of an administration of a cardiopulmonary resuscitation procedure on a heart of a patient in accordance with a rescue protocol including a cardiopulmonary resuscitation protocol and a defibrillating shock protocol.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which can be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as can be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A defibrillation controller (204) for advising an administration of a cardiopulmonary resuscitation procedure on a heart of a patient by a defibrillator (200) in accordance with a rescue protocol including a cardiopulmonary resuscitation protocol and a shock protocol, the cardiopulmonary resuscitation protocol specifying at least one rule/guideline for a guidance by the defibrillator (200) of an administration of a cardiopulmonary resuscitation to the heart of the patient, the shock protocol specifying at least one rule/guideline for a conditional delivery of a defibrillating shock to the heart of the patient by the defibrillator (200), the defibrillation controller (204) comprising:
a non-transitory machine-readable storage medium encoded with instructions for execution by at least one processor, the non-transitory machine-readable storage medium including the instructions to:
during an ongoing execution of the cardiopulmonary resuscitation protocol by the defibrillator (200), determine a probable rescue outcome of the heart of the patient or an improbable rescue outcome of the heart of the patient from a shockable cardiac rhythm of an electrocardiogram of the heart of the patient; and
based on a determination of the probable rescue outcome of the heart of the patient during the ongoing execution of the cardiopulmonary resuscitation protocol by the defibrillator (200), pretimely terminate the ongoing execution of the cardiopulmonary resuscitation protocol by the defibrillator (200) and initiate a succeeding execution of the shock protocol by the defibrillator (200) to deliver the defibrillating shock to the heart of the patient.

2. The defibrillation controller (204) of claim 1, wherein the non-transitory machine-readable storage medium further includes instructions to:
determine the probable rescue outcome of the heart of the patient by deriving one of a current indication or a trending indication of the probable rescue outcome of the heart from the shockable cardiac rhythm of the electrocardiogram of the heart of the patient during the ongoing execution of the cardiopulmonary resuscitation protocol by the defibrillator (200).

3. The defibrillation controller (204) of claim 1, wherein the non-transitory machine-readable storage medium further includes instructions to:
based on a determination of the improbable rescue outcome of the heart of the patient during the ongoing execution of the cardiopulmonary resuscitation protocol by the defibrillator (200), timely terminate the ongoing execution of the cardiopulmonary resuscitation protocol and initiate the succeeding execution of the shock protocol by the defibrillator (200).

4. The defibrillation controller (204) of claim 3, wherein the non-transitory machine-readable storage medium further includes instructions to:
determine the improbable rescue outcome of the heart of the patient by deriving one of a current indication or a trending indication of the improbable rescue outcome of the heart from the shockable algorithm of the heart of the patient during the ongoing execution of the cardiopulmonary resuscitation protocol by the defibrillator (200).

5. The defibrillation controller (204) of claim 3, wherein the non-transitory machine-readable storage medium further includes instructions to:
based on a timely termination of the cardiopulmonary resuscitation protocol and a succeeding execution of the shock protocol by the defibrillator (200), determine the probable rescue outcome of the heart of the patient of the improbable rescue outcome of the heart of the patient from a shockable cardiac rhythm of the electrocardiogram of the heart of the patient during the succeeding execution of the shock protocol by the defibrillator (200); and
control the delivery of the defibrillating shock to the heart of the patient by the defibrillator (200) based on a determination of the probable rescue outcome of the heart of the patient during the succeeding execution of the shock protocol by the defibrillator (200).

6. A defibrillator (200), comprising:
a shock delivery circuit (207) operable to deliver at least one defibrillating shock to a heart of a patient; and
a defibrillation controller (204) for advising an administration of a cardiopulmonary resuscitation procedure on a heart of a patient by a defibrillator (200) in accordance with a rescue protocol including a cardiopulmonary resuscitation protocol and a shock protocol by the defibrillator (200), the cardiopulmonary resuscitation protocol specifying at least one rule/guideline for a guidance by the defibrillator (200) of an administration of a cardiopulmonary resuscitation to the heart of the patient, the shock protocol specifying at least one rule/guideline for a conditional delivery of a defibrillating shock to the heart of the patient by the defibrillator (200),
wherein the defibrillation controller (204) is configured to:
during an ongoing execution of the cardiopulmonary resuscitation protocol by the defibrillator (200), determine a probable rescue outcome of the heart of the patient or an improbable rescue outcome of the heart of the patient from a shockable cardiac rhythm of an electrocardiogram of the heart of the patient; and
based on a determination of the probable rescue outcome of the heart of the patient during the ongoing execution of the cardiopulmonary resuscitation protocol by the defibrillator (200), pretimely terminate the ongoing execution of the cardiopulmonary resuscitation protocol by the defibrillator (200) and initiate a succeeding execution of the shock protocol by the defibrillator (200) to deliver the defibrillating shock to the heart of the patient by the shock delivery circuit (207).

7. The defibrillator (200) of claim 6, wherein the defibrillation controller (204) is further configured to:
determine the probable rescue outcome of the heart of the patient by deriving one of a current indication or a trending indication of the probable rescue outcome from a shockable cardiac rhythm of the electrocardiogram of the heart of the patient during the ongoing execution of the cardiopulmonary resuscitation protocol by the defibrillator (200).

8. The defibrillator (200) of claim 6, wherein the defibrillation controller (204) is further configured to:
based on a determination of the improbable rescue outcome of the heart of the patient during the ongoing execution of the cardiopulmonary resuscitation protocol by the defibrillator (200), timely terminate the ongoing execution of the cardiopulmonary resuscitation protocol and the succeeding execution of the shock protocol by the defibrillator (200).

9. The defibrillator (200) of claim 8, wherein the defibrillation controller (204) is further configured to:
determine the improbable rescue outcome of the heart of the patient by deriving one of a current indication or a trending indication of the improbable rescue outcome of the heart from a shockable algorithm of the heart of the patient during the ongoing execution of the cardiopulmonary resuscitation protocol by the defibrillator (200).

10. The defibrillator (200) of claim 8, wherein the defibrillation controller (204) is further configured to:
based on a timely termination of the cardiopulmonary resuscitation protocol and a succeeding execution of the shock protocol by the defibrillator (200), determine the probable rescue outcome of the heart of the patient or the improbable rescue outcome of the heart of the patient from a shockable cardiac rhythm of the electrocardiogram of the heart of the patient during the succeeding execution of the shock protocol by the defibrillator (200); and
control the delivery of the defibrillating shock to the heart of the patient by the defibrillator (200) based on a determination of the probable rescue outcome of the heart of the patient during the succeeding execution of the shock protocol by the defibrillator (200).

11. A defibrillation method for advising an administration of a cardiopulmonary resuscitation procedure on a heart of a patient by a defibrillator (200) in accordance with a rescue protocol including a cardiopulmonary resuscitation protocol and a shock protocol by the defibrillator (200), the cardiopulmonary resuscitation protocol specifying at least one rule/guideline for a guidance by the defibrillator (200) of an administration of a cardiopulmonary resuscitation to the heart of the patient, the shock protocol specifying at least one rule/guideline for a conditional delivery of a defibrillating shock to the heart of the patient by the defibrillator (200),
the defibrillation method comprising:
during an ongoing execution of the cardiopulmonary resuscitation protocol by the defibrillator (200), determining a probable rescue outcome of the heart of the patient or an improbable rescue outcome of the heart of the patient from a shockable cardiac rhythm of an electrocardiogram of the heart of the patient; and
based on a determination of the probable rescue outcome of the heart of the patient during the ongoing execution of the cardiopulmonary resuscitation protocol by the defibrillator (200), pretimely terminating the ongoing execution of the cardiopulmonary resuscitation protocol by the defibrillator (200) and initiating a succeeding execution of the shock protocol by the defibrillator (200) to deliver a defibrillating shock to the heart of the patient.

12. The defibrillation method of claim 11, wherein the determining of the probable rescue outcome of the heart of the patient includes deriving one of a current indication or a trending indication of the probable rescue outcome from a shockable cardiac rhythm of the electrocardiogram of the heart of the patient during the ongoing execution of the cardiopulmonary resuscitation protocol by the defibrillator (200).

13. The defibrillation method of claim 11, wherein, based on a determination of the improbable rescue outcome of the heart of the patient during the ongoing execution of the cardiopulmonary resuscitation protocol by the defibrillator (200), timely terminating the ongoing execution of the cardiopulmonary resuscitation protocol and initiating the succeeding execution of the shock protocol by the defibrillator (200).

14. The defibrillation method of claim 13, wherein the determining of the improbable the improbable rescue outcome of the heart of the patient by deriving one of a current indication or a trending indication of the improbable rescue outcome of the heart from a shockable algorithm of the heart of the patient during the ongoing execution of the cardiopulmonary resuscitation protocol by the defibrillator (200).

15. The defibrillation method of claim 13, further comprising:
based on a timely termination of the cardiopulmonary resuscitation protocol and a succeeding execution of the shock protocol by the defibrillator (200), determining the probable rescue outcome of the heart of the patient or the improbable rescue outcome of the heart of the patient from a shockable cardiac rhythm of the electrocardiogram of the heart of the patient during the succeeding execution of the shock protocol by the defibrillator (200); and
controlling the delivery of the defibrillating shock to the heart of the patient by the defibrillator (200) based on a determination of the probable rescue outcome of the heart of the patient during the succeeding execution of the shock protocol by the defibrillator (200).
